# EUROPEAN PATENT APPLICATION

(11) **EP 2 436 390 A1**
(43) Date of publication of application: **04.04.2012**
(21) Application number: 11183581.5
(22) Date of filing: 30.09.2011
(51) Int. Cl.: A61K 38/07, A61K 38/08, A61K 38/10, A61K 31/137, A61K 45/06, A61K 47/18, A61K 47/24, A61K 9/00, A61P 23/02

(54) **Topical Anesthetic Uses of Szeto-Schiller Peptides**

(30) Priority: 30.09.2010 US 895000
(71) Applicant: Perricone, Nicholas V., Meriden, CT 06450 (US)
(72) Inventor: Perricone, Nicholas V., Meriden, CT 06450 (US)
(74) Representative: Harding, Charles Thomas

(57) **Abstract**

Topical anesthetic compositions include Szeto-Schiller peptides and a dermatologically acceptable carrier, and optionally a vasoconstrictor and tyrosine. A method of providing local analgesia to the skin involves applying to skin the topical anesthetic compositions.

SEQUENCE LISTING

<110>  Perricone, Nicholas V

<120>  Topical Anesthetic Uses of Szeto-Schiller Peptides

<130>  P045460EP

<150>  US 12/895000
<151>  2010-09-30

<160>  65

<170>  PatentIn version 3.5

<210>  1
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  amidation

<400>  1

Tyr Arg Phe Lys
1


<210>  2
<211>  4
<212>  PRT
<213>  Artificial Sequence

<220>
<223>  chemically synthesized  or purified recombinant, mitochondria
       targeted aromatic-cationic peptide


<220>
<221>  misc-feature
<222>  (1)..(1)
<223>  2',6'-dimethyltyrosine

<220>
<221>  misc-feature
<222>  (2)..(2)
<223>  D-amino acid

<220>
<221>  misc-feature
<222>  (4)..(4)
<223>  amidation

<400>  2

Tyr Arg Phe Lys
1


<210>  3
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  amidation

<400>  3

Phe Arg Phe Lys
1


<210>  4
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (2)..(2)
<223>  2',6'-dimethyltyrosine

<220>
<221>  misc_feature

<222>  (4)..(4)
<223>  amidation

<400>  4

Arg Tyr Lys Phe
1



<210>  5
<211>  4
<212>  PRT
<213>  Artificial Sequence

<220>
<223>  chemically synthesized  or purified recombinant, mitochondria
       targeted aromatic-cationic peptide



<220>
<221>  misc_feature
<222>  (1)..(1)
<223>  2',6'-dimethylphenylalanine

<220>
<221>  misc_feature
<222>  (2)..(2)
<223>  D-amino acid

<220>
<221>  misc_feature
<222>  (4)..(4)
<223>  amidation

<400>  5

Phe Arg Phe Lys
1



<210>  6
<211>  4
<212>  PRT
<213>  Artificial Sequence

<220>
<223>  chemically synthesized  or purified recombinant, mitochondria
       targeted aromatic-cationic peptide



<220>
<221>  misc_feature
<222>  (1)..(1)
<223>  D-amino acid

<220>
<221>  misc_feature
<222>  (2)..(2)
<223>  2',6'-dimethyltyrosine

<220>
<221>  misc_feature
<222>  (4)..(4)
<223>  amidation

<400>  6

Arg Tyr Phe Lys
1


<210>  7
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  2',6'-dimethyltyrosine and amidation

<400>  7

Arg Phe Lys Tyr
1


<210>  8
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (3)..(3)
<223>  2',6'-dimethyltyrosine

<220>
<221>  misc_feature
<222>  (4)..(4)

<223>  amidation

<400>  8

Arg Phe Tyr Lys
1


<210>  9
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (3)..(3)
<223>  2',6'-dimethyltyrosine

<220>
<221>  misc_feature
<222>  (4)..(4)
<223>  amidation

<400>  9

Arg Lys Tyr Phe
1


<210>  10
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  2',6'-dimethyltyrosine and amidation

<400>  10

Arg Lys Phe Tyr
1


<210>  11
<211>  4
<212>  PRT
<213>  Artificial Sequence

<220>
<223>  aromatic-cationic peptide


<220>
<221>  misc_feature
<222>  (3)..(3)
<223>  2',6'-dimethyltyrosine

<220>
<221>  misc_feature
<222>  (4)..(4)
<223>  amidation

<220>
<221>  misc_feature
<222>  (4)..(4)
<223>  D-amino acid and amidation

<400>  11

Phe Lys Tyr Arg
1


<210>  12
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  D-amino acid

<220>
<221>  misc_feature
<222>  (4)..(4)
<223>  amidation

<220>
<221>  misc_feature
<222>  (4)..(4)
<223>  2',6'-dimethyltyrosine and amidation

<400>  12

Phe Lys Arg Tyr
1



<210>  13
<211>  4
<212>  PRT
<213>  Artificial Sequence

<220>
<223>  chemically synthesized  or purified recombinant, mitochondria
       targeted aromatic-cationic peptide



<220>
<221>  misc_feature
<222>  (2)..(2)
<223>  D-amino acid

<220>
<221>  misc_feature
<222>  (3)..(3)
<223>  2',6'-dimethyltyrosine

<220>
<221>  misc_feature
<222>  (4)..(4)
<223>  amidation

<400>  13

Phe Arg Tyr Lys
1



<210>  14
<211>  4
<212>  PRT
<213>  Artificial Sequence

<220>
<223>  chemically synthesized  or purified recombinant, mitochondria
       targeted aromatic-cationic peptide



<220>
<221>  misc_feature
<222>  (2)..(2)
<223>  D-amino acid

<220>
<221>  misc_feature
<222>  (4)..(4)
<223>  2',6'-dimethyltyrosine and amidation

<400>  14

Phe Arg Lys Tyr

1



<210>  15
<211>  4
<212>  PRT
<213>  Artificial Sequence

<220>
<223>  chemically synthesized  or purified recombinant, mitochondria
       targeted aromatic-cationic peptide



<220>
<221>  misc_feature
<222>  (2)..(2)
<223>  2',6'-dimethyltryrosine

<220>
<221>  misc_feature
<222>  (3)..(3)
<223>  D-amino acid

<220>
<221>  misc_feature
<222>  (4)..(4)
<223>  amidation

<400>  15

Phe Tyr Arg Lys
1



<210>  16
<211>  4
<212>  PRT
<213>  Artificial Sequence

<220>
<223>  chemically synthesized  or purified recombinant, mitochondria
       targeted aromatic-cationic peptide



<220>
<221>  misc_feature
<222>  (2)..(2)
<223>  2',6'-dimethyltyrosine

<220>
<221>  misc_feature
<222>  (4)..(4)
<223>  D-amino acid and amidation

<400>  16

Phe Tyr Lys Arg
1

<210>  17
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  2′,6′-dimethyltyrosine

<220>
<221>  misc_feature
<222>  (4)..(4)
<223>  amidation

<400>  17

Lys Phe Tyr Arg
1


<210>  18
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  2′,6′-dimethyltyrosine

<220>
<221>  misc_feature
<222>  (3)..(3)
<223>  D-amino acid

<220>
<221>  misc_feature
<222>  (4)..(4)
<223>  amidation

<400>  18

Lys Tyr Arg Phe
1


<210>  19
<211>  4
<212>  PRT

<213>  Artificial Sequence

<220>
<223>  chemically synthesized  or purified recombinant, mitochondria
       targeted aromatic-cationic peptide


<220>
<221>  misc_feature
<222>  (2)..(2)
<223>  2′,6′-dimethyltyrosine

<220>
<221>  misc_feature
<222>  (4)..(4)
<223>  D-amino acid and amidation

<400>  19

Lys Tyr Phe Arg
1


<210>  20
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  2′,6′-dimethyltyrosine and amidation

<400>  20

Lys Arg Phe Tyr
1


<210>  21
<211>  4
<212>  PRT
<213>  Artificial Sequence

<220>
<223>  chemically synthesized  or purified recombinant, mitochondria
       targeted aromatic-cationic peptide


<220>

<221>  misc_feature
<222>  (2)..(2)
<223>  D-amino acid

<220>
<221>  misc_feature
<222>  (3)..(3)
<223>  2',6'-dimethyltyrosine

<220>
<221>  misc_feature
<222>  (4)..(4)
<223>  amidation

<400>  21

Lys Arg Tyr Phe
1


<210>  22
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (2)..(2)
<223>  2',6'-dimethyltyrosine

<220>
<221>  misc_feature
<222>  (3)..(3)
<223>  D-amino acid

<220>
<221>  misc_feature
<222>  (4)..(4)
<223>  amidation

<400>  22

Arg Tyr Arg Phe
1


<210>  23
<211>  4
<212>  PRT
<213>  Artificial Sequence

<220>
<223>  aromatic-cationic peptide


<220>
<221>  misc_feature
<222>  (1)..(1)
<223>  D-amino acid

<220>
<221>  misc_feature
<222>  (2)..(2)
<223>  2′,6′-dimethyltyrosine

<220>
<221>  misc_feature
<222>  (3)..(3)
<223>  D-amino acid

<220>
<221>  misc_feature
<222>  (4)..(4)
<223>  2′,6′-dimethyltyrosine and amidation

<400>  23

Arg Tyr Arg Tyr
1


<210>  24
<211>  4
<212>  PRT
<213>  Artificial Sequence

<220>
<223>  chemically synthesized  or purified recombinant, mitochondria
      targeted aromatic-cationic peptide


<220>
<221>  misc_feature
<222>  (1)..(1)
<223>  D-amino acid

<220>
<221>  misc_feature
<222>  (2)..(2)
<223>  2′,6′-dimethyltyrosine

<220>
<221>  misc_feature
<222>  (3)..(3)
<223>  D-amino acid

<220>
<221>  misc_feature
<222>  (4)..(4)
<223>  amidation

<400>  24

Arg Tyr Arg Tyr
1


<210>  25
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (2)..(2)
<223>  2′,6′-dimethyltyrosine

<220>
<221>  misc_feature
<222>  (3)..(3)
<223>  D-amino acid

<220>
<221>  misc_feature
<222>  (4)..(4)
<223>  amidation

<400>  25

Arg Tyr Arg Trp
1


<210>  26
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature

<222>  (4)..(4)
<223>  amidation

<400>  26

Trp Arg Phe Lys
1


<210>  27
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  amidation

<400>  27

Trp Arg Tyr Lys
1


<210>  28
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  amidation

<400>  28

Trp Arg Trp Lys
1

<210>  29
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (3)..(3)
<223>  2',6'-dimethyltyrosine

<220>
<221>  misc_feature
<222>  (4)..(4)
<223>  amidation

<400>  29

Trp Arg Tyr Lys
1


<210>  30
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  amidation

<400>  30

Arg Trp Lys Phe
1


<210>  31
<211>  4

<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  amidation

<400>  31

Arg Trp Phe Lys
1


<210>  32
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  2′,6′-dimethyltyrosine and amidation

<400>  32

Arg Trp Lys Tyr
1


<210>  33
<211>  4
<212>  PRT
<213>  Artificial Sequence

<220>
<223>  chemically synthesized  or purified recombinant, mitochondria
       targeted aromatic-cationic peptide

<220>
<221>  misc_feature
<222>  (1)..(1)
<223>  D-amino acid

<220>
<221>  misc_feature
<222>  (3)..(3)
<223>  2',6'-dimethyltyrosine

<220>
<221>  misc_feature
<222>  (4)..(4)
<223>  amidation

<400>  33

Arg Trp Tyr Lys
1


<210>  34
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  amidation

<400>  34

Arg Lys Trp Phe
1


<210>  35
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)

<223>  D-amino acid

<220>
<221>  misc_feature
<222>  (4)..(4)
<223>  2',6'-dimethyltyrosine and amidation

<400>  35

Arg Lys Trp Tyr
1


<210>  36
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  amidation

<400>  36

Ala Arg Phe Lys
1


<210>  37
<211>  4
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  amidation

<400>  37

Trp Lys Tyr Arg
1

<210>  38
<211>  4
<212>  PRT
<213>  Artificial Sequence

<220>
<223>  chemically synthesized  or purified recombinant, mitochondria
       targeted aromatic-cationic peptide

<220>
<221>  misc_feature
<222>  (3)..(3)
<223>  D-amino acid

<400>  38

Tyr His Gly Met
1

<210>  39
<211>  4
<212>  PRT
<213>  Artificial Sequence

<220>
<223>  chemically synthesized  or purified recombinant, mitochondria
       targeted aromatic-cationic peptide

<220>
<221>  misc_feature
<222>  (3)..(3)
<223>  D-amino acid

<400>  39

Phe Arg His Asp
1

<210>  40
<211>  8
<212>  PRT
<213>  Artificial Sequence

<220>
<223>  chemically synthesized  or purified recombinant, mitochondria
       targeted aromatic-cationic peptide

<400>  40

Tyr Arg Phe Lys Glu His Trp Arg
1               5

<210>  41
<211>  6
<212>  PRT
<213>  Artificial Sequence

<220>
<223>  chemically synthesized  or purified recombinant, mitochondria
       targeted aromatic-cationic peptide

<400>  41

Lys Gln Tyr Arg Phe Trp
1               5


<210>  42
<211>  5
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (5)..(5)
<223>  amidation

<400>  42

Tyr Arg Phe Lys Glu
1               5


<210>  43
<211>  5
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  D-amino acid

<400>  43

Met Tyr Lys Phe Arg
1               5


<210>  44
<211>  6
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (5)..(5)
<223>  D-amino acid

<400>  44

His Glu Lys Tyr Phe Arg
1               5


<210>  45
<211>  6
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (5)..(5)
<223>  D-amino acid

<220>
<221>  misc_feature
<222>  (6)..(6)
<223>  amidation

<400>  45

Lys Gln Tyr Arg Phe Trp
1               5

<210>  46
<211>  7
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (6)..(6)
<223>  D-amino acid

<400>  46

Phe Arg Lys Trp Tyr Arg His
1               5


<210>  47
<211>  7
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (6)..(6)
<223>  D-amino acid

<220>
<221>  misc_feature
<222>  (7)..(7)
<223>  amidation

<400>  47

Gly Phe Lys Tyr His Arg Tyr
1               5


<210>  48

<211>  8
<212>  PRT
<213>  Artificial Sequence

<220>
<223>  chemically synthesized  or purified recombinant, mitochondria
       targeted aromatic-cationic peptide



<220>
<221>  misc_feature
<222>  (2)..(2)
<223>  D-amino acid

<220>
<221>  misc_feature
<222>  (5)..(5)
<223>  D-amino acid

<220>
<221>  misc_feature
<222>  (8)..(8)
<223>  amidation

<400>  48

Val Lys His Tyr Phe Ser Tyr Arg
1               5


<210>  49
<211>  8
<212>  PRT
<213>  Artificial Sequence

<220>
<223>  chemically synthesized  or purified recombinant, mitochondria
       targeted aromatic-cationic peptide



<220>
<221>  misc_feature
<222>  (4)..(4)
<223>  D-amino acid

<220>
<221>  misc_feature
<222>  (7)..(7)
<223>  D-amino acid

<400>  49

Trp Lys Phe Asp Arg Tyr His Lys
1               5


<210>  50
<211>  8
<212>  PRT
<213>  Artificial Sequence

<220>
<223>  chemically synthesized  or purified recombinant, mitochondria
      targeted aromatic-cationic peptide


<220>
<221>  misc_feature
<222>  (3)..(3)
<223>  D-amino acid

<220>
<221>  misc_feature
<222>  (8)..(8)
<223>  D-amino acid and amidation

<400>  50

Lys Trp Tyr Arg Asn Phe Tyr His
1               5


<210>  51
<211>  9
<212>  PRT
<213>  Artificial Sequence

<220>
<223>  chemically synthesized  or purified recombinant, mitochondria
      targeted aromatic-cationic peptide


<220>
<221>  misc_feature
<222>  (5)..(5)
<223>  D-amino acid

<220>
<221>  misc_feature
<222>  (8)..(8)
<223>  D-amino acid

<400>  51

Thr Gly Tyr Arg His Phe Trp His Lys
1               5


<210>  52
<211>  9
<212>  PRT
<213>  Artificial Sequence

<220>
<223>  chemically synthesized  or purified recombinant, mitochondria
      targeted aromatic-cationic peptide


<220>
<221>  misc_feature

<222>  (2)..(2)
<223>  D-amino acid

<220>
<221>  misc_feature
<222>  (5)..(5)
<223>  D-amino acid

<220>
<221>  misc_feature
<222>  (8)..(8)
<223>  D-amino acid

<220>
<221>  misc_feature
<222>  (9)..(9)
<223>  amidation

<400>  52

Asp Trp Lys Tyr His Phe Arg Gly Lys
1               5


<210>  53
<211>  10
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(4)
<223>  D-amino acid

<220>
<221>  misc_feature
<222>  (6)..(8)
<223>  D-amino acid

<220>
<221>  misc_feature
<222>  (10)..(10)
<223>  amidation

<400>  53

His Lys Tyr Phe Glu Asp His Lys Arg Trp
1               5                   10

<210>  54
<211>  10
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(3)
<223>  D-amino acid

<220>
<221>  misc_feature
<222>  (6)..(6)
<223>  D-amino acid

<220>
<221>  misc_feature
<222>  (8)..(8)
<223>  D-amino acid

<400>  54

Ala Phe Arg Tyr Lys Trp His Tyr Gly Phe
1               5                   10


<210>  55
<211>  11
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(2)
<223>  D-amino acid

<220>
<221>  misc_feature
<222>  (4)..(4)
<223>  D-amino acid

<220>
<221>  misc_feature
<222>  (7)..(7)
<223>  D-amino acid

<220>
<221>  misc_feature
<222>  (10)..(10)
<223>  D-amino acid

<400>  55

Tyr His Phe Arg Asp Lys Arg His Trp His Phe
1               5                   10


<210>  56
<211>  11
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  D-amino acid

<220>
<221>  misc_feature
<222>  (7)..(7)
<223>  D-amino acid

<220>
<221>  misc_feature
<222>  (9)..(9)
<223>  D-amino acid

<220>
<221>  misc_feature
<222>  (11)..(11)
<223>  amidation

<400>  56

Phe Phe Tyr Arg Glu Asp Lys Arg Arg His Phe
1               5                   10


<210>  57
<211>  12
<212>  PRT
<213>  Artificial Sequence

<220>
<223>  chemically synthesized  or purified recombinant, mitochondria
       targeted aromatic-cationic peptide


<220>
<221>  misc_feature
<222>  (4)..(4)
<223>  D-amino acid

<220>
<221>  misc_feature

<222>  (7)..(8)
<223>  D-amino acid

<220>
<221>  misc_feature
<222>  (11)..(11)
<223>  D-amino acid

<400>  57

Phe Tyr Lys Arg Trp His Lys Lys Glu Arg Tyr Thr
1               5                   10


<210>  58
<211>  12
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  D-amino acid

<220>
<221>  misc_feature
<222>  (6)..(7)
<223>  D-amino acid

<220>
<221>  misc_feature
<222>  (10)..(10)
<223>  D-amino acid

<400>  58

Tyr Asp Lys Tyr Phe Lys Arg Phe Pro Tyr His Lys
1               5                   10


<210>  59
<211>  13
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  D-amino acid

<220>
<221>  misc_feature
<222>  (5)..(5)
<223>  D-amino acid

<220>
<221>  misc_feature
<222>  (7)..(7)
<223>  D-amino acid

<220>
<221>  misc_feature
<222>  (10)..(10)
<223>  D-amino acid

<220>
<221>  misc_feature
<222>  (13)..(13)
<223>  D-amino acid and amidation

<400>  59

Glu Arg Lys Tyr Val Phe His Trp Arg Gly Tyr Arg Met
1               5                   10


<210>  60
<211>  14
<212>  PRT
<213>  Artificial Sequence

<220>
<223>  chemically synthesized  or purified recombinant, mitochondria
       targeted aromatic-cationic peptide


<220>
<221>  misc_feature
<222>  (2)..(3)
<223>  D-amino acid

<220>
<221>  misc_feature
<222>  (7)..(7)
<223>  D-amino acid

<220>
<221>  misc_feature
<222>  (9)..(9)
<223>  D-amino acid

<220>
<221>  misc_feature
<222>  (11)..(11)
<223>  D-amino acid

<220>
<221>  misc_feature
<222>  (13)..(13)
<223>  D-amino acid

<400>  60

Arg Leu Tyr Phe Lys Glu Lys Arg Trp Lys Phe Tyr Arg Gly
1               5                   10


<210>  61
<211>  15
<212>  PRT
<213>  Artificial Sequence

<220>
<223>  chemically synthesized  or purified recombinant, mitochondria
       targeted aromatic-cationic peptide


<220>
<221>  misc_feature
<222>  (1)..(1)
<223>  D-amino acid

<220>
<221>  misc_feature
<222>  (4)..(5)
<223>  D-amino acid

<220>
<221>  misc_feature
<222>  (8)..(8)
<223>  D-amino acid

<220>
<221>  misc_feature
<222>  (12)..(12)
<223>  D-amino acid

<220>
<221>  misc_feature
<222>  (15)..(15)
<223>  amidation

<400>  61

Glu Asp Lys Arg His Phe Phe Val Tyr Arg Tyr Tyr Arg His Phe
1               5                   10                  15


<210>  62
<211>  16
<212>  PRT
<213>  Artificial Sequence

<220>
<223>  chemically synthesized  or purified recombinant, mitochondria
       targeted aromatic-cationic peptide


<220>
<221>  misc_feature

<222>  (3)..(3)
<223>  D-amino acid

<220>
<221>  misc_feature
<222>  (6)..(7)
<223>  D-amino acid

<220>
<221>  misc_feature
<222>  (10)..(10)
<223>  D-amino acid

<220>
<221>  misc_feature
<222>  (12)..(12)
<223>  D-amino acid

<220>
<221>  misc_feature
<222>  (14)..(14)
<223>  D-amino acid

<400>  62

Asp Arg Phe Cys Phe Arg Lys Tyr Arg Tyr Trp His Tyr Phe Lys Phe
1               5                   10                  15


<210>  63
<211>  17
<212>  PRT
<213>  Artificial Sequence

<220>
<223>  chemically synthesized  or purified recombinant, mitochondria
       targeted aromatic-cationic peptide


<220>
<221>  misc_feature
<222>  (3)..(3)
<223>  D-amino acid

<220>
<221>  misc_feature
<222>  (7)..(7)
<223>  D-amino acid

<220>
<221>  misc_feature
<222>  (11)..(11)
<223>  D-amino acid

<220>
<221>  misc_feature
<222>  (14)..(14)
<223>  D-amino acid

<220>

<221>  misc_feature
<222>  (17)..(17)
<223>  amidation

<400>  63

His Tyr Arg Trp Lys Phe Asp Ala Arg Cys Tyr His Phe Lys Tyr His
1               5                   10                  15

Ser

<210>  64
<211>  19
<212>  PRT
<213>  Artificial Sequence

<220>
<223>  chemically synthesized  or purified recombinant, mitochondria
       targeted aromatic-cationic peptide

<220>
<221>  misc_feature
<222>  (5)..(5)
<223>  D-amino acid

<220>
<221>  misc_feature
<222>  (10)..(11)
<223>  D-amino acid

<220>
<221>  misc_feature
<222>  (15)..(15)
<223>  D-amino acid

<220>
<221>  misc_feature
<222>  (18)..(18)
<223>  D-amino acid

<400>  64

Gly Ala Lys Phe Lys Glu Arg Tyr His Arg Arg Asp Tyr Trp His Trp
1               5                   10                  15

His Lys Asp

<210>  65
<211>  20
<212>  PRT
<213>  Artificial Sequence

<220>

<223>  chemically synthesized  or purified recombinant, mitochondria
       targeted aromatic-cationic peptide

<220>
<221>  misc_feature
<222>  (4)..(4)
<223>  D-amino acid

<220>
<221>  misc_feature
<222>  (9)..(10)
<223>  D-amino acid

<220>
<221>  misc_feature
<222>  (13)..(13)
<223>  D-amino acid

<220>
<221>  misc_feature
<222>  (17)..(17)
<223>  D-amino acid

<220>
<221>  misc_feature
<222>  (20)..(20)
<223>  amidation

<400>  65

Thr Tyr Arg Lys Trp Tyr Glu Asp Lys Arg His Phe Tyr Gly Val Ile
1               5                   10                  15

His Arg Tyr Lys
            20

## Description

### FIELD OF THE INVENTION

The present invention relates to topical anesthetic compositions for application to the skin. More specifically, the present invention relates to topical compositions comprising Szeto-Schiller peptides for pain management.

### BACKGROUND OF THE INVENTION

A wide variety of drugs are used in modern anesthetic practice. Anesthetics are categorized into two classes: general, which cause a reversible loss of consciousness, and local, which cause a reversible loss of sensation for a limited region of the body while maintaining consciousness.

Local anesthetics act by binding to fast sodium charnels from within (in an open state). Local anesthetics are typically either ester or amide based compounds. Ester local anesthetics (e.g., procaine, amethocaine, cocaine) are generally unstable in solution and fast-acting, and allergic reactions are common. Amide local anesthetics (e.g., lidocaine, prilocaine, bupivicaine, levobupivacaine, ropivacaine, mepivacaine and dibucaine) are generally heat-stable, with a long shelf life (around 2 years). They have a slower onset and longer half-life than ester anaesthetics, and are usually racemic mixtures, with the exception of levobupivacaine (which is S(-) -bupivacaine) and ropivacaine (S(-)-ropivacaine). These agents are generally used within regional and epidural or spinal techniques, due to their longer duration of action, which provides adequate analgesia for surgery, labor, and symptomatic relief.

A topical anesthetic is a local anesthetic that is used to numb the surface of a body part. They can be used to numb any area of the skin as well as the front of the eyeball, the inside of the nose, ear or throat, the anus and the genital area. Topical anesthetics are available in creams, ointments, aerosols, sprays, lotions, and jellies. Oftentimes, a vasoconstrictor, such as epinephrine, will be combined with a topical anesthetic in order to control bleeding after an injection or to retard diffusion or absorption and prolong the action of the anesthetic agent in the location where it is applied.

Topical anesthetics are also used to numb the outermost surface of the eye in opthalmological and optometric procedures. They are used in dentistry to numb oral tissues before administering a dental local anesthetic and in ear nose and throat (ENT) procedures. Topical anesthetics may be used in connection with other surgical and medical procedures, and also have use in relieving skin irritation. For example, topical anesthetics may be used to relieve pain and itching caused by conditions such as poison ivy, oak or sumac, sunburn or other minor burns, insect bites or stings, and minor cuts and scratches. Topical anesthetics are have also been used as a temporary treatment to prevent premature ejaculation in men.

Szeto and Schiller have disclosed water soluble, highly polar, aromatic cationic peptides for significantly reducing the number of mitochondria undergoing mitochondrial permeability transition (e.g., U.S. Patent No. 7,576,061 B2, incorporated herein by reference) and also for use in therapy as analgesics (U.S. Patent No. 6,703,483; U.S. Patent No. 7,498,297; and U.S. Patent Application Publicaiton No. 2004/0176305 A1, all of which are incorporated herein by reference). The peptides have between two and twenty amino acids covalently joined by peptide bonds.

U.S. 6,703,483 discloses analogs of H-Tyr-D-Arg-Phe-Lys-NH2 (represented by the acronym DALDA, SEQ. ID. NO. 1) for use as analgesics, particularly in obstetrics. The DALDA analogs are taught as mu-opioid agonsists. The "mu" or µ-receptors exist mostly presynaptically in the periaqueductal gray region, and in the superficial dorsal horn of the spinal cord (specifically the substantia gelatinosa of Rolando). Other areas where µ-receptors have been located include the external plexiform layer of the olfactory bulb, the nucleus accumbens, in several layers of the cerebral cortex and in some of the nuclei of the amygdala, as well as the nucleus of the solitary tract. Mu receptors are also found in the intestinal tract. The patent discloses that suitable compositions of DALDA compounds are in liquid form, suitable for administration intrathecally, epidurally, intramuscularly, and intravenously. The antinocicipetide effects of the compounds after intrathecal administration are disclosed, as measured by the tail-flick test in rats

U.S. 7,498,297 also relates to the use of Szeto-Schiller peptides for pain management. Particularly, the patent discloses peptides having two α-amino acid residues, at least one of which has a positive charge, a tyrosine residue in the first position and a D-α-amino acid in the second position for stimulating a mu-opioid receptor. The agonist peptides are taught for administration intrathecally or orally. The antinocicipetide effects of the compounds after administration by injection or intrathecal catheter are disclosed, as measured by the tail-flick test in rats. A method of protecting a mammal's heart from ischemic-repurfusion injury by intravenously administering 2',6'-dimethyl-Tyr-D-Arg-Phe-Lys-NH₂ (Dmt-DALDA, SEQ. ID, NO. 2) is particularly claimed.

In both patents, topical administration is not taught or suggested and the peptides were not applied to the tail for use as a local analgesic before the tail-flick test. Moreover, there is no indication that the compounds are thought to have local anesthetic effects.

Similar to the '483 patent, U.S. 2004/0176305 A1 discloses the possibility of DALDA analgos for use in pain management, particularly during labor. The application speculates that because the peptides bind to opioid receptors, they will activate the receptors and exhibit analgesic activity. Only pharmaceutical compositions in liquid form, suitable for administration intrathecally, epidurally, intramuscularly, and intravenously are disclosed.

Zhao et al., 279 J. Biol. Chem. 34682-34690 (2004), reported that Szeto-Schiller peptides targeted inner the mitochondrial membrane and potently reduced intracellular ROS and cell death caused by tBHP in neuronal cells. In addition, the Szeto-Schiller peptides decreased mitochondrial ROS production, inhibited mitochondrial permeability transition and swelling and prevented cytochrome c release induced by Ca²⁺ in mitochondria. ROS and MPT have been implicated in myocardial stunning, therefore, the inner mitochondrial membrane targeted antioxidants were implicated for treatment of aging and diseases associated with oxidative stress. More recent studies support potential use of Szeto-Schiller peptides for ischemia-reperfusion injury and neurodegenerative disorders. Szeto, 8 AAPS J. E277-E283 (2006).

Most recently, Szeto disclosed that three small, nitrated amino acid sequences carrying a 3+ charge at physiological pH were able to penetrate neuronal and endothelial cells of mice through intravenous administration. Ann. N.Y. Acad. Sci. 1147: 112-121 (2008). In isolated mitochondria, the addition of D-Arg-Dmt-Lys-Phe-NH₂ (SS-31) and Phe-D-Arg-Phe-Lys-NH₂ (SS-20) prevented MPP⁺-induced (1-methyl-4-phenylpyridium) inhibition of oxygen consumption, ATP production, and mitochondrial swelling. Moreover, mice studies showed that SS-31 and SS-20 may be effective in the treatment of Parkinson's Disease (PD) or Amyotrophic Lateral Sclerosis (ALS). The SS peptides were touted as having excellent pharmacokinetic properties and no adverse effects.

Administration of topical anesthetics to control pain associated with medical procedures may avoid the need for infiltrative local anesthesia injections and associated pain from the injections. Topical anesthesia also avoids the risk of wound margin distortion that exists with infiltrative injection administration, Many dosage forms exist (e.g., gels, sprays, creams, ointments, patches) and can provide the clinician with precise options for application under various circumstances.

It is therefore desirable to have topical anesthetic compounds and compositions containing topical anesthetic for use in a variety of applications. It is particularly desirable to have compositions comprising Szeto-Schiller peptides that are suitable for topical administration and may be used as a local anesthetic.

### SUMMARY OF THE INVENTION

The present invention provides topical compositions comprising a carrier and an effective amount of Szeto-Schiller peptide for application to the skin.

Methods for providing local analgesia to the skin comprise applying a composition containing an effective amount of Szeto-Schiller peptide in a dermatologically acceptable carrier to the skin.

More specifically, the present invention provides topical compositions and methods of applying compositions comprising Szeto-Schiller peptide to prevent or treat pain and itching of skin and subdermal areas.

### DETAILED DESCRIPTION OF THE INVENTION

Topical compositions comprising Szeto-Schiller peptides ("SS peptide(s)") are provided for use in pain management. The compositions provide local anesthetic action. Pain management treatments consist of topically applying SS peptides, and optionally a vasoconstrictor and/or tyrosine, to the skin in a dermatologically acceptable carrier.

The term skin, as used herein, means the keratinous surfaces skin, hair and nails. The term skin when used herein is in the broad sense meaning the skin of the face, body, and neck as well as the lips. For purposes of the present invention, the term skin may also include the mucous membranes (or mucosae) continuous with skin: at the nostrils, the mouth, the lips, the eyelids, the ears, the genital area, and the anus.

### Szeto-Schiller Peptides

Szeto-Schiller peptides or "SS peptides" are small, aromatic-cationic, water soluble, highly polar peptides, such as those disclosed in U.S. 6,703,483 and 7,576,061, which can readily penetrate cell membranes. The aromatic-cationic peptides include a minimum of two amino acids, and preferably include a minimum of four amino acids, covalently joined by peptide bonds. The maximum number of amino acids is about twenty amino acids covalently joined by peptide bonds. Optimally, the number of amino acids present in the SS peptides is four.

The amino acids of the aromatic-cationic SS peptides are any amino acid. Preferably, at least one amino group is at the alpha position relative to the carboxyl group. One or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. Carboxyl groups, especially the terminal carboxyl group of a C-terminal amino acid, are preferably amidated with, for example, ammonia to form the C-terminal amide. Alternatively, the terminal carboxyl group of the C-terminal amino acid may be amidated with any primary or secondary amine.

In one embodiment, the amino acid at the N-terminus is phenylalanine or its derivative. Preferred derivatives of phenylalanine include 2'-methylphenylalanine (Mmp), 2',6'-dimethylphenylalanine (Dmp), N,2',6'-trimethylphenylalanine (Tmp), and 2'-hydroxy-6'-methylphenylalanine (Hmp).

The aromatic-cationic peptides have a minimum number of net positive charges at physiological pH in comparison to the total number of amino acid residues in the peptide.

DALDA is a recognized example, as are its functional analogs.

For purposes of the claims, the term "Szeto-Schiller peptide" is defined as the following:
Tyr-D-Arg-Phe-Lys-NH₂(SEQ. ID. NO: 1),
Dmt-D-Arg-Phe-Lys-NH₂(SEQ. ID. NO: 2),
Phe-D-Arg-Phe-Lys-NH₂ (SEQ. ID. NO: 3),
D-Arg-Dmt-Lys-Phe-NH₂ (SEQ. 1D. NO: 4),
Dmp-D-Arg-Phe-Lys-NH₂(SEQ. ID, NO: 5),
D-Arg-Dmt-Phe-Lys-NH₂(SEQ. ID. NO: 6),
D-Arg-Phe-Lys-Dmt-NH₂(SEQ. ID. NO: 7),
D-Arg-Phe-Dmt-Lys-NH₂(SEQ. ID. NO: 8),
D-Arg-Lys-Dmt-Phe-NH₂(SEQ. ID. NO: 9),
D-Arg-Lys-Phe-Dmt-NH₂(SEQ. ID. NO: 10),
Phe-Lys-Dmt-D-Arg-NH₂ (SEQ. ID. NO: 11),
Phe-Lys-D-Arg-Dmt-NH₂(SEQ. ID. NO: 12),
Phe-D-Arg-Dmt-Lys-NH₂(SEQ. ID. NO: 13),
Phe-D-Arg-Lys-Dmt-NH₂(SEQ. ID. NO: 14),
Phe-Dmt-D-Arg-Lys-NH₂(SEQ. ID. NO: 15),
Phe-Dmt-Lys-D-Arg-NH₂(SEQ. ID. NO: 16),
Lys-Phe-Dmt-D-Arg-NH₂(SEQ. ID. NO: 17),
Lys-Dmt-D-Arg-Phe-NN₂(SEQ. ID. NO: 18),
Lys-Dmt-Phe-D-Arg-NH₂(SEQ. ID. NO: 19),
Lys-D-Arg-Phe-Dmt-NH₂(SEQ. ID. NO: 20),
Lys-D-Arg-Dmt-Phe-NH₂(SEQ. ID. NO: 21),
D-Arg-Dmt-D-Arg-Phe-NH₂(SEQ. ID. NO: 22),
D-Arg-Dmt-D-Arg-Dmt-NH₂(SEQ. ID. NO: 23),
D-Arg-Dmt-D-Arg-Tyr-NH₂(SEQ. ID. NO: 24),
D-Arg-Dmt-D-Arg-Trp-NH₂ (SEQ. ID. NO: 25),
Trp-D-Arg-Phe-Lys-NH₂ (SEQ. ID. NO: 26),
Trp-D-Arg-Tyr-Lys-NH₂ (SEQ. ID. NO: 27),
Trp-D-Arg-Trp-Lys-NH₂ (SEQ. ID. NO: 28),
Trp-D-Arg-Dmt-Lys-NH₂ (SEQ. ID. NO: 29),
D-Arg-Trp-Lys-Phe-NH₂ (SEQ. ID. NO: 30),
D-Arg-Trp-Phe-Lys-NH₂ (SEQ. ID. NO: 31),
D-Arg-Trp-Lys-Dmt-NH₂ (SEQ. ID. NO: 32),
D-Arg-Trp-Dmt-Lys-NH₂ (SEQ. ID. NO: 33),
D-Arg-Lys-Trp-Phe-NH₂ (SEQ. ID. NO: 34),
D-Arg-Lys-Trp-Dmt-NH₂ (SEQ. ID. NO: 35),
Cyclohexyl-D-Arg-Phe-Lys-NH₂,
Ala-D-Arg-Phe-Lys-NH₂(SEQ. ID. NO: 36),
Lys-D-Arg-Tyr-NH₂,
Phe-D-Arg-His,
D-Tyr-Trp-Lys-NH₂,
Trp-D-Lys-Tyr-Arg-NH₂ (SEQ. ID. NO: 37),
Tyr-His-D-Gly-Met (SEQ. ID. NO: 38),
Phe-Arg-D-His-Asp (SEQ. ID. NO: 39),
Tyr-Arg-Phe-Lys-Glu-His-Trp-Arg (SEQ. 1D. NO: 40),
Lys-Gln-Tyr-Arg-Phe-Trp (SEQ. ID. NO: 41),
Tyr-D-Arg-Phe-Lys-Glu-NH₂ (SEQ. ID. NO: 42),
Met-Tyr-D-Lys-Phe-Arg (SEQ. ID. NO: 43),
D-His-Glu-Lys-Tyr-D-Phe-Arg (SEQ. ID. NO: 44),
Lys-D-Gin-Tyr-Arg-D-Phe-Trp-NH₂ (SEQ. ID. NO: 45),
Phe-D-Arg-Lys-Trp-Tyr-D-Arg-His (SEQ. ID. NO: 46),
Gly-D-Phe-Lys-Tyr-His-D-Arg-Tyr-NH₂ (SEQ. ID. NO: 47),
Val-D-Lys-His-Tyr-D-Phe-Ser-Tyr-Arg-NH₂ (SEQ. ID. NO: 48),
Trp-Lys-Phe-D-Asp-Arg-Tyr-D-His-Lys (SEQ. ID. NO: 49),
Lys-Trp-D-Tyr-Arg-Asn-Phe-Tyr-D-His-NH₂ (SEQ. ID. NO: 50),
Thr-Gly-Tyr-Arg-D-His-Phe-Trp-D-His-Lys (SEQ. ID. NO: 51),
Asp-D-Trp-Lys-Tyr-D-His-Phe-Arg- D-Gly-Lys-NH₂ (SEQ. ID. NO: 52),
D-His-Lys-Tyr- D-Phe-Glu- D-Asp- D-His- D-Lys-Arg-Trp-NH₂ (SEQ. ID. NO: 53),
Ala-D-Phe-D-Arg-Tyr-Lys-D-Trp-His-D-Tyr-Gly-Phe (SEQ. ID. NO: 54),
Tyr-D-His-Phe- D-Arg-Asp-Lys- D-Arg-His-Trp-D-His-Phe (SEQ. ID. NO: 55),
Phe-Phe-D-Tyr-Arg-Glu-Asp-D-Lys-Arg-D-Arg-His-Phe-NH₂ (SEQ. ID. NO: 56),
Phe-Tyr-Lys-D-Arg-Trp-His-D-Lys-D-Lys-Glu-Arg-D-Tyr-Thr (SEQ. ID. NO: 57),
Tyr-Asp-D-Lys-Tyr-Phe- D-Lys- D-Arg-Phe-Pro-D-Tyr-His-Lys (SEQ. ID, NO: 58),
Glu-Arg-D-Lys-Tyr- D-Val-Phe- D-His-Trp-Arg-D-Gly-Tyr-Arg-D-Met-NH2 (SEQ. ID. NO: 59),
Arg-D-Leu-D-Tyr-Phe-Lys-Glu- D-Lys-Arg-D-Trp-Lys- D-Phe-Tyr-D-Arg-Gly (SEQ. ID. NO: 60),
D-Glu-Asp-Lys-D-Arg-D-His-Phe-Phe-D-Va1-Tyr-Arg-Tyr-D-Tyr-Arg-His-Phe-NH₂ (SEQ. ID. NO: 61),
Asp-Arg-D-Phe-Cys-Phe-D-Arg-D-Lys-Tyr-Arg-D-Tyr-Trp-D-His-Tyr-D-Phe-Lys-Phe (SEQ. ID. NO: 62),
His-Tyr-D-Arg-Trp-Lys-Phe-D-Asp-Ala-Arg-Cys-D-Tyr-His-Phe-D-Lys-Tyr-His-Ser-NH₂ (SEQ. ID. NO: 63),
Gly-Ala-Lys-Phe-D-Lys-Glu-Arg-Tyr-His-D-Arg-D-Arg-Asp-Tyr-Trp-D-His-Trp-His-D-Lys-Asp (SEQ. ID. NO: 64), and
Thr-Tyr-Arg-D-Lys-Trp-Tyr-Glu-Asp-D-Lys-D-Arg-His-Phe-D-Tyr-Gly-Val-lle-D-His-Arg-Tyr-Lys-NH₂ (SEQ. ID. NO: 65).

In a preferred embodiment, the SS peptide has the formula Tyr-D-Arg-Phe-Lys-NH₂ (SEQ. ID. NO: 1, DALDA, which is referred to herein as SS-01). DALDA has a net positive charge of three, contributed by the amino acids tyrosine, arginine, and lysine and has two aromatic groups contributed by the amino acids phenylalanine and tyrosine. In a particularly preferred embodiment, the tyrosine of DALDA can be a modified derivative of tyrosine such as in 2',6'dimethyltyrosine to produce the compound having the formula 2',6'-Dmt-D-Arg-Phe-Lys-NH₂ (SEQ. ID. NO: 2, i.e., Dmt-DALDA, which is referred to herein as SS-02).

In another preferred embodiment, the SS peptide has the formula Phe-D-Arg-Phe-Lys-NH₂ (sect. ID. NO: 3, i.e., Phe⁻¹-DALOA, which is referred to herein as SS-20). In another embodiment, the amino acid sequence of Dmt¹⁻DALDA (SS-02) is rearranged such that Dmt is not at the N-terminus, such as the formula D-Arg-2',6'-Dmt-Lys-Phe-NH₂ (SEQ. ID. NO: 4, referred to in this specification as SS-31).

### Synthesis of Peptides

The peptides useful in the methods of the present invention may be chemically synthesized by any of the methods well known in the art. Suitable methods for synthesizing the protein include, for example those described by Stuart and Young in "Solid Phase Peptide Synthesis," Second Edition, Pierce Chemical Company (1984), and in "Solid Phase Peptide Synthesis," Methods Enzymol. 289, Academic Press, Inc, New York (1997).

### Compositions and Methods

A particular object of the present invention is to provide SS peptides to enhance skin penetration and transdermal absorption. The SS peptides are small and contain an amino acid sequence that allows them to freely penetrate cells, enabling the compounds of the invention to be effective as a topical application that can easily pass through the lipid bilayer of the cell membranes of epidermal and dermal cells. They are taken up into cells in an energy-independent nonsaturable manner. When having a D-amino acid in either the first or second position, they are resistant against aminopeptidase activity, and amidiation of the C-terminus reduces hydrolysis from the C-terminus.

Topical compositions containing Szeto-Schiller peptides according to the present invention are intended to be topically applied to and absorbed by the skin tissue. The compositions can be used to anesthetize the skin before intramuscular injection, venipuncture, and simple skin procedures such as curettage or biopsy. Procedures such as laceration repair can be accomplished safely and comfortably with the compositions of the present invention. An additional advantage of the compositions of the present invention is that wound margins are not distorted.

Only effective amounts of topical compositions containing SS peptide(s) are needed to achieve the aforementioned properties. The quantity of SS peptide active ingredient in the carrier may be varied or adjusted widely depending upon the particular application, the potency of the particular compound or the desired concentration.

In one embodiment, the quantity of SS peptide active ingredient will range between 10 ppm to 2500 ppm, and preferably 100 ppm to 1000 ppm.

In alternate embodiments, the quantity of SS peptide active ingredient will range between 0.0001% to 0.1 % by weight. In different embodiments, the weight percentage of SS peptide may be in the range of 0.005% - 0.0025%; 0.0025% - 0.005%; 0.005% - 0.01 %; 0.01% - 0.02%; 0.02% - 0.03%; 0.03% - 0.04%; 0,04% - 0.05%; or 0.05% - 0.1 %. Generally, lower concentrations of SS peptide active ingredients in a carrier are suitable, depending upon the application regimen and the active and adjunct ingredients employed. Preferably, the peptide comprises up to 0.1% by weight of the composition.

In some applications, the quantity of SS peptide active ingredient may exceed 0.1 % by weight. For example, in some embodiments, the quantity of SS peptide active ingredient will be in the range between 0.01 % to 5% w/w; or in the range from 0.05% to 4% w/w; or in the range from 0.05% to 1% w/w.

However, the preferred embodiment includes the SS peptide active ingredient in the range from 100 ppm to 1000 ppm. Most preferably, the SS peptide is in the range from 500 to 1000 ppm.

In one embodiment of the invention, the SS-peptide may be administered along with a vasoconstrictor. Epinephrine is a common and preferred example although any vasoconstrictor employed by those of skill in the art is contemplated. The vasoconstrictor allows a reservoir of the SS peptide to be located and stored in the upper layers of skin during application. The amount of epinephrine may be about 0.01 to about 0.5 % w/w of the composition. Preferably, the amount of epinephrine is about 0.05 to about 0.1% w/w.

In another embodiment, the composition further comprises tyrosine. In dopaminergic cells in the brain, tyrosine is converted to levodopa by the enzyme tyrosine hydroxylase (TH). TH is the rate-limiting enzyme involved in the synthesis of the neurotransmitter dopamine. In addition, in the adrenal medulla, tyrosine is converted into the catecholamine hormones norepinephrine (noradrenaline), and epinephrine. Thus, the addition of tyrosine may also allow a reservoir of the SS peptide to be located and stored in the upper layers of skin during application, thereby enhancing the effect of the peptide. The tyrosine may be present in a composition up to 100 ppm.

In yet another embodiment of the invention, the composition may be compounded with other commonly known topical anesthetics e.g. tetracaine, lidocaine, benzocaine, procaine, etc.

While not wishing to be bound by any theory, it is believed that the SS peptides will provide a numbing sensation upon contact by preventing the initiation and transmission of nerve impulses and providing cutaneous analgesia by targeting free nerve endings in the dermis. It is believed that the tyrosine residues of certain peptides, e.g. SS-02 and SS-31, provide an enhanced localized effect because tyrosine is a precursor to neurotransmitters and increases plasma neurotransmitter levels. Upon absorption, the SS peptides may bind to the mu-opioid receptors and provide additional anesthetic relief, even after removal of the topical composition from the skin or eye.

Antioxidant activity of SS peptides used the present invention can be attributed to the tyrosine or dimethyltyrosine (Dmt) residue. Tyrosine can scavenge oxyradicals forming relatively unreactive tyrosyl radicals, which can be followed by radical-radical coupling to give dityrosine, or react with superoxide to form tyrosine hyperoxide. Dimethyltyrosine is more effective than tyrosine in scavenging ROS. The specific location of the tyrosine or dimethyltyrosine residue is not as important as SS-31 was found to be as effective as SS-02 in scavenging H₂O₂ and inhibiting LDL oxidation. However, replacement of Dmt with phenylalanine (SS-20) eliminated the scavenging ability.

Onset of action, anesthesia depth, and duration of action are determined by the pKa level, pH level, lipid solubility, protein binding, and vasodilatory effects of the specific peptide used. These factors also depend on the area of the skin to which the anesthetic is applied, the vascularity of tissues, and the surface area of the skin or eye.

Generally, topical application to skin tissue is accomplished in association with a dermatologically acceptable carrier, and particularly one in which the SS peptide is soluble per se or is effectively solubilized (e.g., as an emulsion or microemulsion). Where employed, the carrier is inert in the sense of not bringing about a deactivation or oxidation of the SS peptide, and in the sense of not bringing about any adverse effect on the skin areas to which it is applied.

In one preferred practice of the invention, one or more SS peptides is applied in admixture with the dermatologically acceptable carrier or vehicle (e.g., as a lotion, cream, ointment, soap, stick, or the like) so as to facilitate topical application and, in some cases, provide additional therapeutic effects as might be brought about, e.g., by moisturizing of the affected skin areas. While the carrier for the topical composition can consist of a relatively simple solvent or dispersant such as water, it is generally preferred that the carrier comprise a composition more conducive to topical application, and particularly one which will form a film or layer on the skin to which it is applied so as to localize the application and provide some resistance to washing off by immersion in water or by perspiration and/or aid in the percutaneous delivery of the active agent(s). Many preparations are known in the art, and include lotions containing oils and/or alcohols and emollients vegetable oils, hydrocarbon oils and waxes, silicone oils, animal or marine fats or oils, glyceride derivatives, fatty acids or fatty acid esters, or alcohols or alcohol ethers, lecithin, lanolin and derivatives, polyhydric alcohols or esters, wax esters, sterols, phospholipids and the like, and generally also emulsifiers (nonionic, cationic or anionic), although some of the emollients inherently possess emulsifying properties. In the preferred embodiment, the carrier is lecithin.

A liposomal carrier may also be as a vehicle for delivering the SS peptides. Liposomes are comprised of lipid layers surrounded by aqueous layers. They are able to penetrate the stratum corneum because they resemble the lipid layers of the cell membrane and can provide sustained release. Liposomes as drug carriers also protect the anesthetic from metabolic degradation, allowing prolonged duration of action.

As noted, these ingredients can be formulated into a cream, lotion, or gel, or a solid stick, by utilization of different proportions of the ingredients and/or by inclusion of thickening agents such as gums or other forms of hydrophilic colloids. One possible embodiment is a solution used to saturate a pad or wipe used to apply the compounds to affected areas; another is a cleanser; and others are lotions, creams, and gels, which are referred to herein as dermally or dermatologically acceptable carriers, and are formulated using conventional techniques known to those of ordinary skill in the art. Another possible embodiment is a cream which, as it dries, becomes a flexible membrane that is easily removed i.e. a peel. Anesthetic patches, with or without the use of controlled heating, and use of formulations with iontophoresis are also contemplated.

The topical composition of the invention can contain additional ingredients commonly found in skin care compositions, such as, for example, tinting agents, emollients, skin conditioning agents, emulsifying agents, humectants, preservatives, antioxidants, perfumes, chelating agents, etc., provided that they are physically and chemically compatible with other components of the composition. Preservatives include, but are not limited to, C₁-C₃ alkyl parabens and phenoxyenthanol, typically present in an amount ranging from about 0.1 % to about 2.0% by weight percent, based on the total composition. Emollients, typically present in amounts ranging from about 0.01% to 5% of the total composition include, but are not limited to, fatty esters, fatty alcohols, mineral oils, polyether siloxane copolymers, and mixtures thereof. Humectants, typically present in amounts ranging from about 0.1 % to about 5% by weight of the total composition include, but are not limited to, polyhydric alcohols such as glycerol, polyalkylene glycols (e.g., butylene glycol, propylene glycol, dipropylene glycol, polypropylene glycol, and polyethylene glycol) and derivatives thereof, alkylene polyols and their derivatives, sorbitol, hydroxy sorbitol, hexylene glycol, 1,3-dibutylene glycol, 1,2,6-hexanetriol, ethoxylated glycerol, propoxylated glycerol, and mixtures thereof. Emulsifiers, typically present in amounts from about 1% to about 10% by weight of the composition, include, but are not limited to, stearic acid, cetyl alcohol, stearyl alcohol, steareth 2, steareth 20, acrylates/C₁₀₋₃₀ alkyl acrylate crosspolymers, and mixtures thereof. Chelating agents, typically present in amounts ranging from about 0.01 % to about 2% by weight, include, but are not limited to, ethylenediamine tetraacetic acid (EDTA) and derivatives and salts thereof, dihydroxyethyl glycine, tartaric acid, and mixtures thereof. Antioxidants, typically present in an amount ranging from about 0.02% to about 0.5% by weight of the composition, include, but are not limited to, butylated hydroxy toluene (BHT); vitamin C and/or vitamin C derivatives, such as fatty acid esters of ascorbic acid, particularly ascorbyl palmitate; butylated hydroanisole (BHA); phenyl-a-naphthylamine; hydroquinone; propyl gallate; nordihydroquiaretic acid; vitamin E and/or derivatives of vitamin E, including tocotrienol and/or tocotrienol derivatives; calcium pantothenates; green tea extracts; mixed polyphenols; and mixtures of any of these. Particularly preferred antioxidants are those that provide additional benefits to the skin such as ascorbyl palmitate.

Buffering agents are employed in many compositions. Preferably, the amount of buffering agent is one that results in compositions having a pH ranging from about 4.5 to about 8.5, more preferably from about 5.5 to about 8.5, most preferably from about 6.5 to about 8.0. Typical buffering agents are chemically and physically stable agents commonly found in cosmetics, and can include compounds that are also adjunct ingredients such as citric acid, malic acid, and glycolic acid buffers.

Some embodiments of this invention contain at least one other adjunct ingredient in addition to the SS peptide active ingredient. Adjunct ingredients include, but are not limited to one or more of: lipoic acid; a-hydroxy acids such as glycolic acid or lactic acid; ascorbic acid and its derivatives, especially fatty acid esters of ascorbic acid; or tocotrienols and tocotrienol derivatives and vitamin E compositions enriched with tocotrienols or tocotrienol derivatives. Additional ingredients and methods as disclosed in my U.S. Patent Nos. 5,376,361; 5,409,693; 5,545,398; 5,554,647; 5,574,063; 5,643,586; 5,709,868; 5,879,690; 6,191,121; 6,296,861; 6,437,004; and 6,979,459, which are hereby incorporated by reference, may also be used.

The term "topical composition" as used in the following description shall mean the complete product including the SS peptide active ingredient, and any vasoconstrictor, tyrosine, carrier, adjuvants, thickeners, excipients, etc. as described herein which is applied to a person's skin.

Generally in the practice of methods of the invention, the topical composition will topically applied to the skin areas where there is pain or where pain will occur as the result of a dermatological or medical procedure. When in solution form, 1 to 5 mL of solution may be directly applied to a wound by placing a few drops in the wound. A cotton tipped applicator may then be applied with pressure maintained for 15 to 40 minutes. Another option is to apply a thick layer of a more viscous mixture to intact skin. After application, the area may be covered with a patch of Tegaderm or clear plastic wrap to facilitate penetration through the stratum corneum.

When working near mucous membranes, a gel is better than a solution because it stays within the wound. When using compositions containing epinephrine or tyrosine, application to end-arteriolar parts of the body, such as the digits, should be avoided.

In a preferred method, the topical compositions are used to decrease pain associated with laser pulses, surgical procedures, or soft tissue augmentation, such as that performed by a dermatologist.

It is an advantage of the invention that compositions of the invention do not require a pharmaceutical prescription.

The above description is for the purpose of teaching the person of ordinary skill in the art how to practice the present invention, and it is not intended to detail all those obvious modifications and variations of it which will become apparent to the skilled worker upon reading the description. It is intended, however, that all such obvious modifications and variations be included within the scope of the present invention, which is defined by the following claims. The claims are intended to cover the claimed components and steps in any sequence which is effective to meet the objectives there intended, unless the context specifically indicates the contrary.

## Claims

1. A topical anesthetic composition comprising an effective amount of at least one Szeto-Schiller peptide and a dermatologically acceptable carrier.

2. The topical anesthetic composition of claim 1, comprising:
an effective amount of at least one Szeto-Schiller peptide selected from the group consisting of: Tyr-D-Arg-Phe-Lys-NH2 (SEQ. ID. NO: 1), Dmt-D-Arg-Phe-Lys-NH2 (SEQ. ID. NO: 2), Phe-D-Arg-Phe-Lys-NH2 (SEQ. ID. NO. 3) and D-Arg-Dmt-Lys-Phe-NH2 (SEQ ID. NO: 4);
a dermatologically acceptable skin -penetrating carrier;
a vasoconstrictor; and
tyrosine.

3. The composition of claim 2 wherein the peptide is SEQ. ID. NO: 2.

4. The composition of claims 1, 2, or 3 wherein the composition includes from about 0.0001 % w/w to about 0.1% w/w peptide.

5. The composition of claims 1, 2, or 3, wherein the composition includes from about 0.01% w/w to about 5% w/w peptide.

6. The composition of claims 1, 2, or 3, wherein the composition includes from about 100 ppm to 1000 ppm peptide.

7. The composition of claims 1, 2, 3, 4, 5, or 6 wherein the carrier comprises lecithin.

8. The composition of claim 2 wherein the vasoconstrictor is epinephrine.

9. The composition of claim 2 wherein the tyrosine is present up to 100 ppm.

10. A method of providing local analgesia to the skin comprising: applying to the skin tissue of a mammal in need of such regulation, a safe and effective amount of a composition comprising at least one Szeto-Schiller peptide and a dermatologically acceptable carrier.

11. The method of claim 10 wherein the Szeto-Schiller peptide is selected from the group consisting of: Tyr-D-Arg-Phe-Lys-NH₂ (SEQ. ID. NO: 1), Dmt-D-Arg-Phe-Lys-NH₂ (SEQ. ID. NO: 2), Phe-D-Arg-Phe-Lys-NH₂ (SEQ. ID. NO. 3) and D-Arg-Dmt-Lys-Phe-NH₂ (SEQ ID. NO: 4).

12. The method of claim 11, wherein the peptide is SEQ. ID. NO: 2.
